# EUROPEAN PATENT APPLICATION

(11) **EP 2 070 510 A1**
(43) Date of publication of application: **17.06.2009**
(21) Application number: 07301654.5
(22) Date of filing: 10.12.2007
(51) Int. Cl.: A61K 8/11, A61Q 19/10, A47K 7/02

(54) **Personal cleansing system**

(71) Applicant: Takasago International Corporation, Tokyo-to 144-8721 (JP)
(72) Inventor: Warr, Jonathan, 75017 Paris (FR); Aussant, Emmanuel, 75011 Paris (FR); Fraser, Stuart, Cheshire - CH64 3 DH (GB)
(74) Representative: Gillard, Marie-Louise

(57) **Abstract**

The present invention relates to a personal cleansing system comprising:
(1) a scrubbing device containing at least one hydrophobic synthetic polymer;
(2) a liquid personal cleansing composition containing core shell capsules in which are encapsulated benefit agents.

Application: personnal cleansing.

## Description

### Technical Field

This invention is related to the field of personal cleansing, especially the use of scrubbing devices, in conjunction with liquid personal cleansing products which contain capsules. More particularly, this invention is related to the use of scrubbing devices which break more of the capsules present in liquid skin cleansing products containing soap or surfactants or mixtures thereof whilst bathing or showering.

### Description of the Prior Art

Personal cleansing formulations have to satisfy many requirements some of which may even be contradictory. They need to clean effectively and economically but they must also be mild and non irritating to the skin even upon frequent use and increasingly they need to deliver aesthetic sensory experiences during and subsequent to the cleansing process. Product manufacturers have developed a wide range of liquid personal cleansing formulations, some specialised to specific tasks such as eye make-up removal, others for general skin cleansing to be used while showering or bathing. This invention relates to the latter group of liquid cleansing products for use while bathing or showering. Liquid shower and bathing products may also be designed for particular groups of people e.g. baby wash products or shower products which contain skin moisturisers. It is common for manufacturers to introduce additives to their cleansing products, both as an aid to cleansing as in abrasive exfoliating particles, or to repair damage to the skin adding for example plant extracts such as chamomile oil or to provide additional aesthetic benefit agents such as moistening oils or fragrances. An increasingly popular way of delivering benefit agents from liquid cleansing products is to encapsulate the benefit agents so that they are separated from the cleansing ingredients. There are numerous advantages to separating the benefit agents in terms of stability on storage and for better targeted delivery and release.

It is known to combine personal wash products with scrubbing devices for various purposes as described in US patent 5,650,384, which is concerned with improved lathering or US patent 5,916,586 which describes a polymeric diamond mesh scrim scrubbing device used in conjunction with a liquid cleansing formulation containing a deodorant fragrance. Neither describes the use of capsules as part of the personal cleansing composition.

US patent application 2005/226900 describes fragrance containing capsules for incorporation into liquid personal cleansing products such as body washes shower gels and shampoos but does not allude to breaking the capsules during the cleansing process. Indeed the example indicates that the intention is for the capsules to be deposited intact onto the skin and to be released either by towelling or subsequent mechanical action. Again no mention is made of the use of scrubbing devices as a way to improve the efficiency of capsule rupture, nor that the capsule should be broken during the cleansing process.

While large capsules can be broken by rubbing the product on the skin with the hand it is far more difficult to break small capsules dispersed in aqueous systems simply by rubbing with the hand as noted in US patent 6,231,873 which states that it is more difficult to break small capsules than larger ones and that below 50 µm it may not be possible to break capsules just with hand pressure. In order for capsules to remain uniformly distributed throughout a liquid product requires that ideally neutral buoyancy should be maintained between the capsules and liquid. From the density difference of the capsules and surrounding liquid and the liquid viscosity (the more viscous the liquid the more slowly a capsule will cream or settle) a maximum capsule particle size can be calculated which will remain dispersed. In general capsules containing hydrophobic benefit agents need to be below 50 µm average particle diameter.

Therefore, there is a need of means to break capsules efficiently whilst bathing or showering.

It has now been found that the capsules may be efficiently broken whilst bathing or showering by using a scrubbing device.

Thus it is the object of this invention to claim the use of scrubbing devices to break capsules containing fragrances or other agents, present in personal cleansing products, during the cleansing process.

### Summary of the invention

The present invention relates to the use of scrubbing devices in order to break capsules more efficiently during personal washing to release the ingredients within capsules in liquid personal cleansing products.

Also claimed is personal cleansing system comprising:
(1) a scrubbing device containing at least one hydrophobic synthetic polymer;
(2) a liquid personal cleansing composition containing core shell capsules in which are encapsulated benefit agents.

The core shell capsules can be broken by the scrubbing device during showering or bathing to release the encapsulated benefit agent or agents.

The invention also relates to a process for cleansing the skin comprising applying on the skin a liquid personal cleansing composition containing core shell capsules in which are encapsulated benefit agents and using a scrubbing device to release the benefit agents on the skin.

### Detailed Description of the Invention

In the following specification, all percentages quoted are weight percent unless otherwise stated and all documents cited in this specification are incorporated herein by reference.

The present invention relates to the use of scrubbing devices to break capsules (containing benefit agents) present in personal cleansing liquids more efficiently than can be done with the hand during bathing or showering. Both the scrubbing device and liquid cleansing product of the invention are intended for frequent use as part of the regular general domestic cleansing of the body during showering or bathing. The invention is not meant to cover specialist products designed for cleansing particular areas of the body such as eyes or feet, nor of products designed as products for skin treatments which would be carried out less frequently than personal washing.

### Scrubbing Devices

A wide variety of devices have been used in combination with personal cleansing products such as bar soaps or liquid products to assist in cleansing and refreshing the skin by removing dirt and dead skin from the body whilst showering or bathing. The cleansing device, herein termed a scrubbing device, although that term is not intended to imply any particular harshness or abrasive quality of the device, nor any particular type of use. Scrubbing devices of the invention have a three dimensional structure which may have been created from a two dimensional sheet structure in manufacture but using multiple folds or loops to create a three dimensional cleansing device. Examples of such devices include terry towel wash cloths or mitts, brushes, various cleaning pads, made from natural or synthetic materials such as natural sponges or loofahs. or synthetic sponges as described in US patent 4,627,129 or as can be purchased from retail outlets. The important feature of the scrubbing device is that it maintains the ability to break capsules present in the liquid cleansing product when wet. Many natural hydrophilic materials such as natural sponges or cotton or linen devices soften in water. The ability of the scrubbing device to break capsules is partly a property of the materials employed and partly due to construction. Scrubbing devices made from hydrophobic synthetic polymers maintain their ability to break capsules even when wet whereas many scrubbing devices made from natural hydrophilic polymers particularly those that are knitted or woven to create the scrubbing device such as a terry towelling mitt soften to such an extent that they do not break capsules very efficiently when wet. Hence a preferred form of scrubbing device comprises a significant or predominant proportion of a hydrophobic, synthetic polymer. Many forms of construction are possible, for example a mitt may contain 50% of a synthetic polymer which could be that one side of the mitt is entirely synthetic polymer while the other face is made from a different material such as cotton. Mitts having two differently textured surfaces are commercially available. Alternatively a mitt may be made from a textile made from a mixture of fibres so that the hydrophobic synthetic polymer is uniformly distributed throughout the mitt. Scrubbing devices may comprise devices made from textured film as described in US patent 6,957,924. Preferred forms of scrubbing device comprises a hydrophobic polymeric fibre synthetic sponge as described in US patent 5,144,744 or US patent 5,709,434 which describes a different device construction but still based on hydrophobic synthetic polymer mesh. Puffs or synthetic sponges of the invention have an aspect ratio of less than 100:1 for the ratio of longest to shortest dimensions; preferably this ratio is less than 25:1 and more preferably less than 10:1 for the device in a dry and uncompressed state. The devices are not thin sheets as commonly used for wet wipes. Preferred devices also comprise more than 50% of voids within the body of the device, preferably more than 70% of voids within the device and more preferably greater than 85% of voids within the device. By voids is meant that the device is a network of polymer filaments creating interconnecting air spaces within the body of the device. Also if the device is submerged in water it displaces considerably less than its uncompressed 3-dimensional volume, preferably less than 50% of its uncompressed volume more preferably less than 10% of its uncompressed volume. Moreover it is preferred if the device is comprised of more than 25%, preferably more than 50% and even more preferably more than 75% of one or more hydrophobic synthetic polymers. Particularly suitable hydrophobic synthetic polymers include polyethylene, polypropylene and ethyl vinyl acetate copolymers. Especially preferred are scrubbing devices made using predominantly (by weight) polyethylene or polypropylene mesh. It is also preferred if the fibres of the mesh have a diameter greater than 1 µm more preferably that they have a diameter greater than 25 µm and even more preferably that they have a diameter greater than 100 µm, i.e. they are not comprised of microfibres. Examples of scrubbing devices for skin cleansing are listed in the following patents, all of which are incorporated herein by reference: US 3,343,196; US 5,727,278; FR 2,287,886; US 6,957,924.

### Liquid personal cleansing products

According to the present invention many personal care compositions may be used to deliver the benefit containing capsules. Examples include shower gels, liquid soaps, shampoos, foaming bath oils etc. The liquid personal cleansing compositions for use in the invention are intended for frequent use for general skin cleansing during showering or bathing.

Typical personal cleansing products would preferably comprise:
(1) from about 3% to about 95%, preferably from about 3% to about 85%, more preferably from about 3% to about 30%, even more preferably from about 5% to about 25%, and most preferably from 10 to 25%, of a surfactant system;
(2) 0.001% to about 10% preferably from about 0.01% to 5% more preferably 0.01% to about 2% and even more preferably from about 0.01% to about 1.0% of encapsulated benefit agents contained within capsules and
(3) optionally a thickening agent and minor ingredients such as colorant, sequestrant, preservative and unencapsulated fragrance;
(4) the balance comprising liquid carrier, normally comprising material selected from the group consisting of water; C₁-C₄ monohydric alcohols; C₂-C₆ polyhydric alcohols; propylene carbonate; liquid polyalkylene glycols; and the like; and mixtures thereof.

Preferably the surfactant system would comprise one or more ingredients which are known to be mild to the skin, and the liquid carrier is water.

The formulations and ingredients of personal cleansing products to which encapsulated ingredients may be added are well known to those skilled in the art, reference may be made to the following works:
"Formulating Detergents and Personal Care Products A Guide to Product Development" by L Ho Tan Tai, ISBN 1-893997-10-3 published by the AOCS Press, and "Harry's Cosmeticology" published by CHS Press 8th Ed. 2000 ISBN 0-8206-0372-4, as well as to the following patents and patent applications: US 4,491,539; US 4,540,507; US 4,565,647; US 5,804,539; US 5,849,310; US 7,098,180; US 2005/226900; US 2005/227907.

### Surfactants

Numerous examples of surfactants are disclosed in the patents incorporated herein by reference. They include anionic surfactants, nonionic surfactants, cationic surfactants, amphoteric surfactants, zwitterionic surfactants, and mixtures thereof. They include alkyl sulfates, alkylpolyethyleneglycol sulfates, alkyl sulfonates, alkyl glyceryl ether sulfonates, anionic acyl sarcosinates, methyl acyl laurates, N-acyl glutamates, acyl isethionates, alkyl sulfosuccinates, alkyl phosphate esters, ethoxylated alkyl phosphate esters, protein condensates, mixtures of ethoxylated alkyl sulfates and alkyl amine oxides, betaines, sultaines, and mixtures thereof. The hydrophobic alkyl, chains for the surfactants are normally C₈-C₂₂, preferably C₁₀-C₁₈. Further examples of surfactants can be found in McCutcheon's Detergents and Emulsifiers published by Allured.

### Optional Ingredients

### Optional Thickening Agent

The present compositions may well have a high viscosity in order to suspend capsules more easily. Viscosity modification can be achieved in numerous ways for example by manipulating the surfactant microstructure or by including a thickening polymer or suspending agent into the composition or by a combination of these techniques. Any viscosity modifying agent will be used at an effective level for suspending capsules or other materials. The suspension should, in general, be stable for at least one month at ambient temperature. Longer term shelf stability, such as at least three months, preferably six months, most preferably at least about twenty-four months, is preferred. In general, the compositions used in the present invention will comprise from about 0.01% to about 10%, by weight, of a suspending agent or combination of suspending agents.

One type of suspending agent is a network suspending agent which is preferably present in the liquid personal cleansing compositions used in the present invention at a level of about 0.5% to about 5%, more preferably about 1% to about 4%, most preferably about 1% to about 3%. Such suspending agents are described in US patent 5,849,310.

Preferred network suspending agents are acyl derivatives and amine oxides, especially acyl derivatives, especially those which can be solubilized in a premix solution and then form networks upon cooling. These materials will comprise long chain (e.g. C₈-C₂₂ preferably C₁₄-C₂₂, more preferably C₁₆-C₂₂) aliphatic groups, i.e., long chain acyl derivative materials and long chain amine oxides, as well as mixtures of such materials. Included are ethylene glycol long chain esters, alkanol amides of long chain fatty acids, long chain esters of long chain fatty acids, glyceryl long chain esters, long chain esters of long chain alkanolamides, and long chain alkyl dimethyl amine oxides, and mixtures thereof.

Suitable suspending agents for use herein include ethylene glycol esters of fatty acids preferably having from about 14 to about 22 carbon atoms, more preferably 16-22 carbon atoms. More preferred are the ethylene glycol stearates, both mono and distearate, but particularly the distearate containing less than about 7% of the mono stearate. Other suspending agents include alkanol amides of fatty acids, preferably having from about 16 to about 22 carbon atoms, more preferably about 16 to 18 carbon atoms. Preferred alkanol amides are stearic monoethanolamide, stearic diethanolamide, stearic monoisopropanolamide and stearic monoethanolamide stearate. Other long chain acyl derivatives include long chain esters of long chain fatty acids (e.g. stearyl stearate, cetyl palmitate); glyceryl esters (e.g. glyceryl distearate) and long chain esters of long chain alkanol amides (e.g. stearamide, diethanolamide distearate, stearamide monoethanolamide stearate). Ethylene glycol esters of long chain carboxylic acids, long chain amine oxides and alkanol amides of long chain carboxylic acids can be used as suspending agents in addition to the preferred materials listed above.

Suspending agents also include long chain amine oxides such as alkyl (C₁₆-C₂₂) dimethyl amine oxides, e.g., stearyl dimethyl amine oxide. If the compositions contain an amine oxide or a long chain acyl derivative which is a surfactant, the suspending function could also be provided by such amine oxide or acyl derivative, provided at least a portion of them are present in network form and additional suspending agents may not be needed.

The network suspending agent can be incorporated into the liquid personal cleansing product hereof by solubilizing it into a solution containing water and the anionic sulfate surfactant at a temperature above the melting point of the suspending agent. The suspending agent is then recrystallized, typically by cooling the solution to a temperature sufficient to induce the network formation.

### Optional Polymeric Suspending Agents, Thickeners and Viscosity Modifiers

Polymeric thickeners are categorized as cationic, nonionic or anionic and are selected to provide the desired viscosities. Suitable thickeners are listed in the Glossary and Chapters 3, 4, 12 and 13 of the Handbook of Water-Soluble Gums and Resins, Robert L. Davidson, McGraw-Hill Book Co. , New York, N. Y., 1980.

Anionic thickeners include crosslinked polymers. These crosslinked polymers typically contain one or more monomers derived from acrylic acid, substituted acrylic acids, and salts and esters of these acrylic acids and the substituted acrylic acids, wherein the crosslinking agent contains two or more carbon-carbon double bonds and is derived from a polyhydric alcohol. The preferred polymers for use herein are of two general types. The first type of polymer is a crosslinked homopolymer of an acrylic acid monomer or derivative thereof (e.g. wherein the acrylic acid has substituents on the two and three carbon positions independently selected from the group consisting of C₁-C₄ alkyl, -CN, -COOH, and mixtures thereof). The second type of polymer is a crosslinked copolymer having a first monomer selected from the group consisting of an acrylic acid monomer or derivative thereof (as just described in the previous sentence), a short chain alcohol (i.e. a C₁-C₄) acrylate ester monomer or derivative thereof (e.g. wherein the acrylic acid portion of the ester has substituents on the two and three carbon positions independently selected from the group consisting of C₁-C₄ alkyl, -CN, -COOH, and mixtures thereof), and mixtures thereof and a second monomer which is a long chain alcohol (i. e. C₈-C₄₀) acrylate ester monomer or derivative thereof (e.g. wherein the acrylic acid portion of the ester has substituents on the two and three carbon positions independently selected from the group consisting of C₁-C₄ alkyl, -CN, -COOH, and mixtures thereof). Combinations of these two types of polymers are also useful herein.

In the first type of crosslinked homopolymers, the monomers are preferably selected from the group consisting of acrylic acid, methacrylic acid, ethacrylic acid and mixtures thereof, with acrylic acid being most preferred. In the second type of crosslinked copolymers the acrylic acid monomer or derivative thereof is preferably selected from the group consisting of acrylic acid, methacrylic acid, ethylacrylic acid, and mixtures thereof, with acrylic acid, methacrylic acid, and mixtures thereof being most preferred. The short chain alcohol acrylate ester monomer or derivative thereof is preferably selected from the group consisting of C₁-C₄ alcohol acrylate esters, C₁-C₄ alcohol methacrylate esters, C₁-C₄ alcohol ethacrylate esters and mixtures thereof, with the C₁-C₄ alcohol acrylate esters, C₁-C₄ alcohol methacrylate esters and mixtures thereof, being most preferred. The long chain alcohol acrylate ester monomer is selected from C₈-C₄₀ alkyl acrylate esters, with C₁₀-C₃₀ alkyl acrylate esters being preferred.

The crosslinking agent in both of these types of polymers is a polyalkenyl polyether of a polyhydric alcohol containing more than one alkenyl ether group per molecule, wherein the parent polyhydric alcohol contains at least 3 carbon atoms and at least 3 hydroxyl groups. Preferred crosslinkers are those selected from the group consisting of allyl ethers of sucrose and allyl ethers of pentaerythritol and mixtures thereof. These polymers useful in the present invention are more fully described in following patents US 5,087,445; US 4,509,949; US 2,798,053. See also, CTFA International Cosmetic Ingredient Dictionary, fourth edition, 1991, pp. 12 and 80; which are also incorporated herein by reference.

Other examples of anionic commercially available homopolymers useful herein include the carbomers, which are homopolymers of acrylic acid crosslinked with allyl ethers of sucrose or pentaerythritol. The carbomers are available as the Carbopol® 900 series from B. F. Goodrich. Examples of commercially available copolymers of the second type useful herein include copolymers of C₁₀-C₃₀ alkyl acrylates with one or more monomers of acrylic acid, methacrylic acid, or one of their short chain (i.e. C₁-C₄ alcohol) esters, wherein the crosslinking agent is allyl ether of sucrose or pentaerythritol. These copolymers are known as acrylates/C₁₀-C₃₀ alkyl acrylate crosspolymers and are commercially available as Carbopol® 1342, Pemulen TR-1, and Pemulen TR-2, from B. F. Goodrich. Other optional copolymers of acrylic acid crosslinked with polyallyl sucrose are provided by B. F. Goodrich Company as, for example, Carbopol 934, 940, 941, and 956. A carboxyvinyl polymer is an interpolymer of a monomeric mixture comprising a monomeric olefinically unsaturated carboxylic acid and from about 0.01% to about 10% by weight of the total monomers of a polyether of a polyhydric alcohol, which polyhydric alcohol contains at least four carbon atoms to which are attached at least three hydroxyl groups, the polyether containing more than one alkenyl group per molecule. Other monoolefinic monomeric materials can be present in the monomeric mixture if desired, even in predominant proportion. Carboxyvinyl polymers are substantially insoluble in liquid, volatile organic hydrocarbons and are dimensionally stable on exposure to air.

Preferred polyhydric alcohols used to produce carboxyvinyl polymers include polyols selected from the class consisting of oligosaccharides, reduced derivatives thereof in which the carbonyl group is converted to an alcohol group, and pentaerythritol; more preferred are oligosaccharides, most preferred is sucrose. It is preferred that the hydroxyl groups of the polyol which are modified be etherified with allyl groups, the polyol having at least two allyl ether groups per polyol molecule. When the polyol is sucrose, it is preferred that the sucrose have at least about five allyl ether groups per sucrose molecule. It is preferred that the polyether of the polyol comprise from about 0.01% to about 4% of the total monomers, more preferably from about 0.02% to about 2.5%.

Preferred olefinically unsaturated carboxylic acids for use in producing carboxyvinyl polymers used herein include polymerizable, alpha-beta monoolefinically unsaturated lower aliphatic carboxylic acids; more preferred are monomeric monoolefinic acrylic acids of the structure:
CH₂ =C(R)-COOH, where R is a substituent selected from the group consisting of hydrogen and lower alkyl groups; most preferred is acrylic acid.

Preferred carboxyvinyl polymers used in formulations of the present invention have a molecular weight of at least about 750,000; more preferred are carboxyvinyl polymers having a molecular weight of at least about 1,250, 000; most preferred are carboxyvinyl polymers having a molecular weight of at least about 3,000,000.

The anionic cellulosic thickeners can also include carboxymethyl cellulose and the like.

Nonionic cellulosic thickeners include, but are not limited to: 1-hydroxyethylcellulose; 2-hydroxymethylcellulose; 3-hydroxypropylcellulose; and/or 4-hydroxybutylmethylcellulose.

A suitable thickener is hydroxyethylcellulose, e.g. Natrosol. RTM. 250 KR sold by The Aqualon Company.

Other thickeners useful herein include acrylated steareth-20 methylacrylate copolymer sold as Acrysol® ICS-1 by Rohm and Haas Company; the carboxylic polymers disclosed in US patent 5,318,774; inorganic salts, i.e. chloride, sulfates, etc., at a level of from about 0.1% to about 5%, preferably from about 0.5% to about 3%; and fatty acids and fatty alcohols at a level of from about 1% to about 15%, preferably from about 2% to about 10%.

The liquid personal cleansing products can be thickened by using polymeric additives that hydrate, swell or molecularly associate to provide body (e. g., hydroxypropyl guar gum).

Liquid personal cleansing products can be made with from about 0.1% to about 5%, preferably from about 0.3% to about 3%, of a cationic polymer, having a molecular weight of from about 1,000 to about 5,000,000, especially those selected from the group consisting of:
(I) cationic polysaccharides;
(II) cationic copolymers of saccharides and synthetic cationic monomers, and
(III) synthetic polymers selected from the group consisting of:
   - (A) cationic polyalkylene imines;
   - (B) cationic ethoxy polyalkylene imines; and
   - (C) cationic poly[N-[-3-(dimethylammonio)propyl]-N'-[3-(ethylene-oxyethylene dimethylammonio)propyl]urea dichloride].

Other materials which can also be used as optional suspending agents include those that can impart a gel-like viscosity to the composition, such as water soluble or colloidally water soluble polymers like cellulose ethers (e.g. hydroxyethylcellulose), guar gum, polyvinyl alcohol, polyvinyl pyrrolidone, hydroxypropyl guar gum, starch and starch derivatives, and other thickeners, viscosity modifiers, gelling agents, etc. Mixtures of these materials can also be used.

Another type of suspending agent that can be used is xanthan gum. Xanthan gum is biosynthetic gum material that is commercially available. It is a heteropolysaccharide with a molecular weight of greater than 1 million. It is believed to contain D-glucose, D-mannose and D-glucuronate in the molar ratio of about 2.8:2.0:2.0. The polysaccharide is partially acetylated with about 4.7% acetyl. This information and other is found in Whistler, Roy L. Editor Industrial Gums--Polysaccharides and Their Derivatives New York: Academic Press, 1973. Kelco, a Division of Merck & Co., Inc., offers xanthan gum as Keltrol. RTM.

In general, the level of optional suspending agent and other viscosity modifiers should preferably be as low as possible to achieve the benefit for which the material is added. Optional suspending agent thickeners and viscosity modifiers, etc., when used are in general used at a level of from about 0.01% to about 10%, most commonly from about 0.02% to about 5.0%, preferably from about 0.1% to about 2%, and more preferably from about 0.2% to about 1.0% by weight of the total composition.

### Water

The personal cleansing compositions of the present invention typically comprise from about 40% to about 89%, preferably from about 50% to about 85%, more preferably from about 60% to about 80%, by weight, of water.

The pH of the neat personal cleansing compositions herein is generally not critical and can be from 3.0 to about 9.5, preferably from about 4 to about 8 and more preferably from about 5 to about 8 as measured at 25° C.

The personal cleansing composition preferably has a viscosity of from about 100 cps to about 60,000 cps at 25° C, preferably 500cps to 30,000 cps and more preferably from about 2,000 cps to about 20,000 cps. The term "viscosity" as used herein means the viscosity as measured by a Brookfield RVTDCP with a spindle CP-41 at 1 RPM for 3 minutes, unless otherwise specified. The "neat" viscosity is the viscosity of the undiluted liquid cleanser.

### Benefit agents to be encapsulated

A benefit agent in the context of the current invention is any material which gives a pleasant sensory experience whilst showering or bathing. So the experience might be an olfactory fragrance benefit, a hygiene benefit, tactile benefit from a skin softening material, a warming, cooling or tingling effect from a chemaesthetic material, an emotive or mood enhancing benefit from a psychological or physiological relaxing agent such as soluble natural oil. Preferably the benefit is an olfactory one due to the release of a fragrance composition from the capsules. It may also be possible to combine these effects within a single product either by pre-mixing different benefit agents within a single emulsion prior to encapsulation or by mixing capsules which contain different core materials.

In the context of this specification a perfume composition, which may also be termed a perfume or a fragrance composition, or a fragrance, means any mixture, i.e. more than one chemical species, including materials which act as malodor counteractants. A perfume composition is particularly preferred as the benefit agent, and comprises 50% to 100%, preferably 60% to 100%, and even more preferably 70% to 100% and especially preferably 80% to 100% by weight of the benefit agents. The physical parameters of suitable perfume compositions are described in EP 1767185A1.A wide variety of odiferous materials are known for perfumery use, including materials such as alkenes, alcohols, aldehydes, ketones, esters, ethers, nitriles, amines, oximes, acetals, ketals, thiols, thioketones, imines, etc. Without wishing to be limited, the ingredients of the perfume composition will have molecular weights of less than 325 atomic mass units, preferably less than 300 atomic mass units and more preferably less than 275 atomic mass units to ensure sufficient volatility to be noticeable when the capsules release. Furthermore the perfume compounds will have molecular weights greater than 100 atomic mass units, preferably greater than 120 atomic mass units as lower masses may be too volatile or too water soluble. Ingredients of the fragrance compositions will not contain strongly ionizing functional groups such as sulphonates, sulphates or quaternary ammonium ions.

Naturally occurring plant and animal oils and exudates comprising complex mixtures of various chemical components are also known for use as perfumes, and such materials can be used herein. The principal chemical components of most naturals are known (within ranges), and thus for the most part they can be assessed in the same way as synthetic aroma chemicals. Perfume compositions of the present invention can be relatively simple in their composition with a minimum of two perfume or fragrance ingredients or can comprise highly complex mixtures of natural and synthetic chemical components, chosen to provide any desired odour. It is preferred if the perfume composition contains more than 3 components, more preferable if they contain more than 5 components and even more preferred if they contain more than 8 components. Perfume ingredients are described more fully in S. Arctander, Perfume Flavors and Chemicals. Vols. I and II, Montclair, N.J., and the Merck Index, 8th Edition, Merck & Co., Inc. Rahway, N.J.

Since it is inherent in the success of this invention that more fragrance will be deposited on skin and that the local concentration around ruptured capsules will be quite high, the composition of the capsule core must take into account the less desirable characteristics of some fragrance materials such as allergenic or irritant effects with some humans.

In general, since the capsules will deliver fragrance more efficiently to the surface fewer capsules and hence less fragrance is needed to achieve a desired fragrance effect, so the overall environmental load is reduced. However the greater concentration on skin or in close proximity to the skin requires additional care to formulate the core composition using only ingredients known to be safe in such a context. Among the materials known to have undesirable characteristics and therefore preferably excluded from the perfume compositions of the invention are nitro musks, as exemplified by musk ambrette (CAS 83-66-9), and musk ketone (CAS 81-14-1). Solvents especially the phthalate esters and carbitol ethers defined as R-(OCH₂CH₂)ₙ-OR¹ where n= 1, 2 or 3 R= (C₁-C₇) alkyl or phenyl or alkyl substituted phenyl and R¹ is H or (C₁-C₇)alkyl. Materials listed in Annex 1 of the Dangerous Substances Directive (67/548/EEC) or any of its amendments or ATPs (Adaptation to Technical Progress), or classified as R43 in their safety data sheet are optionally restricted to less than 1% of the core composition, preferably less than 0.1% by weight, more preferably below 0.001%, and even more preferably below the analytical detection limit.

It is also preferable if the levels of the following ingredients are limited to less than 25% by weight, preferably less than 10% by weight and more preferably less than 1% by weight of the core composition: Amyl cinnamic aldehyde 122-40-7, Amyl cinnamic alcohol 101-85-9, Anisyl alcohol 105-13-5, Benzyl alcohol 100-51-6, Benzyl benzoate 120-51-4, Benzyl cinnamate 103-41-3, Benzyl salicylate 118-58-1, Cinnamic aldehyde 104-55-2, Cinnamyl alcohol 104-54-1, Citronellal 106-22-9, Coumarin 91-64-5, Eugenol 97-53-0, Farnesol 4602-84-0, Geraniol 106-24-1, Hexyl cinnamic aldehyde 101-86-0, Hydroxycitronellal 107-95-5, Hydroxymethylpentylcyclihexenecarboxaldehyde 31906-04-4, Isoeugenol 97-54-1, Lilial 80-54-6, Limonene 5989-27-5, Linalool 78-70-6, Methyl heptine carbonate 111-12-6, 3-methyl-4-(2,6,6-trimethyl-2-cyclohexen-1-yl)-3-buten-2-one 127-51-5

### Other Benefit Agents

In the context of this specification, other benefit agents means any material capable of being encapsulated in the way described later and which can deliver a benefit agent when used in a liquid personal cleansing product.

Benefit agents include natural extracts or materials which have therapeutic effects as relaxants or stimulants, e.g. 1,3,5-trimethoxybenzene (621-23-8), 1,3-dimethoxy-5-methylbenzene, or vitamins or vitamin derivatives such as tocopheryl acetate (58-95-7) or retinyl palmitate (79-81-2) are also benefit agents within this definition. Materials which suppress or reduce malodour and its perception by any of the many mechanisms proposed are benefit agents as described in US patents 4,622,221 and US 4,719,105. Materials which when added to the emulsion improve the properties of the core emulsion before encapsulation, or the properties of the capsules themselves. Materials which provide a warming or cooling effect such as described in Cosmetics and Toiletries Vol. 120 No 5 p105 by M Erman are also benefit agents. Examples of such agents include but are not limited to: cyclohexane carboxamide N-ethyl-5-methyl-2-(1-methylethyl) known as WS3^{™} (39711-79-0); menthyl lactate (59259-38-0); (-)-menthoxypropane 1,2-diol known as cooling agent 10^{™}. Materials which act as insect repellents p-menthane-3,8-diol (42822-86-6) or natural plant oils such as Tea Tree oil, Neem oil or eucalyptus oil are benefit agents. Materials which act as antioxidants such as butylated hydroxyl toluene (128-37-0) or butylated hydroxyanisole (25013-16-5) or pentaerythrityl tetra- dit-butyl hydroxyhydrocinnamate, octadecyl di t-butyl-4-hydroxyhydrocinnamate (2082-79-3), tetrabutyl ethylidenebisphenol (35958-30-6) are benefit agents. Materials which act as UV absorbers such as Benzophenone, 3-butylmethoxydibenzoylmethane or Tetrakis-[methylene-3-(3',5'-di-tert-butyl-4'-hydroxyphenyl)propionate]methane (6683-19-8), bis-ethylhexyloxyphenolmethoxyphenyl-triazine are benefit agents. The materials listed above are intended to exemplify the benefit agents but are not intended to limit the benefit agents to this list. Mixtures of the above may also be considered as benefit agents of the invention.

### Capsules

An essential part of the invention is core shell capsules which contain the benefit agent and are broken by the scrubbing device during washing, showering, or bathing. The capsules of the invention are preferably core shell capsules which comprise a central core of water insoluble material surrounded by a water insoluble polymeric layer, preferably the core of the capsule should be predominantly comprised of perfume, surrounded by a polymeric layer which forms the wall or shell layer. The word shell may give the impression of a hard walled capsule but this need not necessarily be so, it indicates no more than a covering layer which protects the contents; so the shell may be plastic and deformable. Capsules of the invention are typically 1-1000 µm in diameter; preferably 2-500 µm and more preferably 5-100 µm. Suitable monomers for core shell capsules are for example methacrylate esters as exemplified in international application WO 01/49817, and urethanes as exemplified in international application WO 03/099005. These materials can either be used as the sole monomer or in admixture with other monomers depending on the desired capsule properties. Additional suitable monomers are well known to those skilled in the art of polymerisation reactions.

One form of core shell capsule is those typically formed by coacervation techniques. The materials and processes are described in detail in the following patents US 2,800,458; US 3,159,585; US 3,533,958; US 3,697,437; US 3,888,689; US 3,996,156; US 3,965,033; US 4,010,038; and US 4,016,098. Preferred encapsulating materials are gelatin coacervated with a polyanion such as gum arabic and more preferably cross-linked with a cross-linking material such as glutaraldehyde, or alginates co-acervated with calcium ions.

### Aminoplast Capsules

A preferred form of capsules is aminoplast capsules formed by condensation polymerization of amines and aldehydes preferably melamine with formaldehyde and optionally containing urea. Patents describing compositions and processes for manufacturing aminoplast capsules in the form of a dispersion are EP patent application 1,246,693 A1 and US patent 6,261,483. Since the capsule is prepared by a reaction of aldehyde and amine it is reasonable that any aldehyde or amine components of the benefit agents might react and so not be available in the capsule core. So it is desirable that the benefit agents contain little or no aldehyde compounds, including alpha, beta unsaturated aldehydes, or primary or secondary amines. Without wishing to limit the patent in any way a typical process for preparing an aminoplast capsule dispersion would include the following steps.

The preparation of an emulsion of the perfume ingredients and any benefit agents or modifiers which may include emulsifying agents or emulsion stabilizers takes place under vigorous agitation.

The first step is the addition of fragrance oil, methylated melamine-formaldehyde resin (with a melamine:formaldehyde:methanol mixture in the approximate molar ratios 1:3:2 to 1:6:4) and an emulsifier. These monomers may be precondensed or the monomers may be used directly. Some of the melamine can be replaced by urea.

Acid is added to adjust to a pH of 3.5 to 6.5 and the temperature raised to 30-45°C. Agitation is allowed to proceed until the dispersion is oil free. Any acid which has no adverse properties may be used in this process, such as for example formic acid or acetic acid.

It is particularly advantageous if the capsules are cured by heating to a temperature between 60°C to 100°C for several hours under moderate agitation.

It is particularly advantageous if during the early phase of curing a further addition of urea, melamine or other amines, or mixtures thereof can be made to reduce the formaldehyde concentration in the finished dispersion and increase the wall thickness. Typically 10-30% additional melamine and/or urea can be added at this stage and a particularly advantageous ratio is 5:1 to 1:1 melamine:urea.

Once curing is complete, the temperature is reduced to around 50°C, and the dispersion is neutralized before being adjusted to a pH around 8.5.

For ease of handling or to modify the viscosity when introducing capsules into liquid products, it is preferable to add a dispersing agent to the slurry to prevent the capsules sedimenting or creaming which may form large aggregates which can be difficult to redistribute.

The final capsule dispersion should preferably contain less than 0.3% by weight preferably less than 100 ppm (wt/wt) and more preferably less than 10 ppm (wt/wt) of free formaldehyde. Any art-accepted method may be used to determine the amount of free formaldehyde in the capsule dispersion. Methods may include the EPA method EPA 8315A, Determination of Carbonyl Compounds by High Performance Liquid Chromatography, or High-Performance Liquid Chromatographic Determination of Free Formaldehyde in Cosmetics Preserved with Dowicil 200, Journal of Chromatography, 502 (1990), pages 193-200.

A variation of this procedure is described in US patent application 2007/0138674 which describes the preparation of a core shell capsule in which the shell wall comprises other polymers as well as a formaldehyde and melamine.

It may also be advantageous to incorporate physically or chemically further materials to improve capsule deposition to substrates such as skin or the the scrubbing device or to improve deposition selectivity during application.

Capsules of the above process will generally have an average particle diameter within the range from 1-500 µm, preferably within the range 5-100 µm and more preferably within the range 10-70µm depending on the emulsifying conditions. The capsule wall will have an average thickness within the range 0.025-1.0 µm. These parameters are preferable for the proper functioning of the capsules of this invention.

The final dispersion may typically contain from 2.5%-80%, preferably 10%-70% and more preferably 20%-70% capsules dispersed in water.

### Method of Use for Personal Cleansing Compositions

The present compositions are used in a conventional manner for cleansing the skin and/or the body and to provide benefits, such as olfactory aesthetic benefit. An effective amount of the composition, typically from about 1 g to about 15 g of the composition, is applied to the body that has preferably been wetted, generally with water. Application to the body includes dispensing of the composition onto the hand, onto the body, or onto a scrubbing device rubbing the device onto the skin to apply the composition, rupture the capsules and release the contents. The product is rinsed with water and if desired the washing procedure can be repeated.
The present invention will be now described in more details by the following non limiting examples.

### Example 1: Composition of fragrance

| Ingredient | CAS number | Weight % |
|---|---|---|
| Camphor Gum Powder | 76-22-2 | 8.0 |
| Carvone L | 6485-40-1 | 15.0 |
| Delta Damascone | 57378-68-4 | 2.0 |
| Dihydromyrcenol | 18479-58-8 | 36.0 |
| Dynascone* | 56973-85-4 | 1.0 |
| Eucalyptol | 470-82-6 | 15.0 |
| Beta Ionone | 14901-07-6 | 6.0 |
| Ethyl 2-methylpentanoate | 39255-32-8 | 1.0 |
| Menthone G | 89-80-5 | 9.0 |
| Triplal** | 27939-60-2 | 7.0 |

| | | |
|---|---|---|
| * Dynascone is a registered trade mark of Firmenich et Cie. ** Triplal is a registered trade mark of International Flavors and Fragrances. | | |

### Example 2: Capsule Preparation

A 21 cylindrical stirring vessel was fitted with a height adjustable disperser having a standard commercial dispersion disk with a diameter of 50 mm.
The vessel was charged in succession with:
- 241 g of the fragrance oil of example 1;
- 43 g of a 70% strength aqueous solution of a methylated melamine-formaldehyde resin (molar ratio melamine: formaldehyde: methanol 1:3.9:2.4) with a viscosity of 275 mPas and a pH of 8.5;
- 38 g of a 20% strength solution of poly-2-acrylamido-2-methylpropane sulfonic acid sodium salt;
- 191 g of water;
- 8.6 g of 10% strength by weight aqueous formic acid solution.

This charge was processed to a capsule dispersion by adjusting the stirring speed to a peripheral speed of approximately 20 mps. The temperature was held at about 35°C.

After 60 minutes, the dispersion was oil-free; a particle size of about 30-50 µm had been established. The stirring speed of the dispersion disk was then reduced to a level sufficient for uniform circulation of the vessel contents.

A cure temperature of 80°C was set, and once reached a feed of a 27% suspension of melamine-urea (ratio 2.5:1, melamine: urea) in formic acid (to adjust pH to pH 4.5) was added to the dispersion of the preformed capsules with a constant mass flow rate and was metered in over the course of an hour. A total of 25 g of the suspension of melamine-urea was metered in. A cure phase of 120 min ensues at 90°C.After the dispersion had been cooled to about 55°C, it was neutralized with triethanolamine to pH 7.0 and adjusted to a pH of 8.5 using ammonia. A dispersing agent was added to give a uniform capsule dispersion with solids content of about 50% and a viscosity of 83mPas.

Analysis of the final dispersion showed the majority of capsules to be in the range of 35-40 µm diameter. The dispersion to contain 37.7% encapsulated fragrance and 0.33% free fragrance.

### Example 3: Unfragranced Shower Gel

The following formulation was used in examples 5 to 8.

| Ingredient | Weight % | Supplier |
|---|---|---|
| Deionised water | 44.9 | |
| Acculyn 22* | 2.00 | Rohm & Haas |
| Glycerin | 3.00 | |
| Sodium Lauryl Sulphate | 30.00 | |
| Dehyton K COS** | 15.00 | Cognis |
| Plantacare 1200UP** | 5.00 | Cognis |
| Merguard 1200*** | 0.1 | Nalco |
| Deionised water | To 100% | |

| | | |
|---|---|---|
| * registered trade mark of Rohm and Haas of Philadelphia USA ** registered trade mark of Cognis GmbH and Co KG Monheim Germany *** registered trademark of Nalco illinois USA | | |

The Acculyn 22 was dispersed in the water under moderate agitation. Then, the other ingredients in order are added under stirring until each is dissolved.

### Example 4: a Typical Thickened Liquid Personal Cleansing Product

| Ingredients | Weight % |
|---|---|
| Sodium Lauryl sulphate | 17.0 |
| Disodium Cocoamphodiacetate | 2.0 |
| C₁₀-C₃₀ alkyl acrylate copolymer | 0.3 |
| Polyquaternium 7 | 1.4 |
| Cocamide MEA | 0.4 |
| PEG-150 Distearate | 0.25 |
| Tetrasodium EDTA | 0.25 |
| Preservative | q.s. |
| Citric acid/sodium hydroxide | q.s for pH 5.5 to 6.0 |
| Water | To 100% |

### Example 5 Capsule Breakage Test

2.05 g of the capsule dispersion of example 2 containing the fragrance of example 1 was added into 50.05 g a shower gel base of example 3 and mixed thoroughly. 1.5 g of the mixture was applied by dropping pipette onto: a 100% polypropylene puff, a cotton towelling wash mitt each of the devices had been soaked in water at 30°C for 5 minutes and also onto a prewetted hand acting as a control. The control sample applied in the hand was rubbed 3 times along a pre-wetted forearm then the shower gel sample was then scraped off the hand and forearm using a spatula and collected into a petri dish. The puff was squeezed in the hand for 10 seconds, each of the mitts was folded over and the two sides rubbed against each other for 10 seconds. All four samples were placed on aluminium foil and covered with a dome 19 cm in diameter having a central opening of 6 cm. Seven trained panelists scored perfume intensity using a 0 to 5 interval scale with 5 being the highest strength and 0 being undetectable fragrance. The table lists the average panel score for each treatment.

| | Puff¹ | Cotton Mitt² | Polypropylene Mitt³ | Hand |
|---|---|---|---|---|
| Mean Score | 4.6 | 1.3 | 2.6 | 1.6 |

| | | | | |
|---|---|---|---|---|
| 1 The puff was purchased from Monoprix S.A. of Clichy France and is made entirely from polypropylene mesh with individual fibres of about 160 µm diameter. It is approximately spherical in shape having a diameter of 12 cm in uncompressed form and weighing 50.5 g. Similar devices are available from many pharmacies, supermarkets and stores selling personal care products. 2 The cotton mitt is made from 100% cotton towelling fabric having dimensions of 20cm x14cm x1cm and weighing about 37g when dry under ambient conditions. Individual threads have a diameter of about 640 µm. Again these mitts are available from many pharmacies, supermarkets and stores selling personal care products. 3 The 75% polyethylene 25% cotton wash mitt is constructed from polyethylene fibres of about 180 µm diameter looped through a thin cotton fabric base. The mitt weighed 37g and had dimensions of 22cm x 12cm x 1cm. The mitt was purchased from Monoprix S.A of Clichy France but similar devices are available from many pharmacies, supermarkets and stores selling personal care products. Clearly the puff was most successful at breaking the capsules and releasing the contents whilst the softer wet cotton towelling mitt and the hand were less successful. | | | | |

### Example 6

This example demonstrates that different scrubbing devices break capsules to different degrees. To 50 g of shower gel base of example 2 was added 1.25 g of capsules of example 1 which were slowly mixed to distribute the capsules. This dispersion was applied to two identical scrubbing devices using a dropping pipette to deliver 2.5 g of sample to each device distributed as evenly as possible. One sample of each pair was rubbed or manipulated in the hand for 5 seconds to break the capsules just before presentation to the perfume intensity assessment panel. Again the whole experiment from mixing the capsule dispersion to completion of the sensory testing was completed in less than 1 hour. The panel scored perfume intensity on a 0-5 scale. The mean intensity scores are shown in the table below.

| Scrubbing Device | Rubbed Score | Non Rubbed Score |
|---|---|---|
| Puff 100% polypropylene | 4.6 | 0.9 |
| Mitt (75% polyethylene 25% cotton) | 4.0 | 1.7 |
| White 100% cotton towelling mitt | 2.5 | 0.8 |

From the scores it is evident that scrubbing devices with a higher proportion of synthetic polymer break more of the capsules consequently releasing more fragrance.

### Example 7

A sample of 1.0 g of the shower gel of example 2 containing capsules of example 1 was dropped onto the scrubbing device or into a wetted hand then the devices were rubbed along the skin of the forearm. The skin is rinsed and gently patted dry with a paper towel and the perfume on skin assessed by the panel.

| | Puff | Cotton Mitt | (75/25) Polyethylene/cotton Mitt | Hand |
|---|---|---|---|---|
| Mean Score | 3.7 | 2.3 | 3.8 | 2.5 |

Again the puff and polypropylene mitt shows greater fragrance remaining on skin.

## Claims

1. A personal cleansing system comprising:
(1) a scrubbing device containing at least one hydrophobic synthetic polymer;
(2) a liquid personal cleansing composition containing core shell capsules in which are encapsulated benefit agents.

2. The personal cleansing system according to claim 1, in which the scrubbing device contains more than 25% by weight of hydrophobic synthetic polymers

3. The personal cleansing system according to either of claim 1 or 2, wherein the scrubbing device comprises multiple folds of hydrophobic synthetic polymers.

4. The personal cleansing system according to any one of claims 1 to 3, wherein the scrubbing device comprises multiple folds of polyethylene and/or polypropylene mesh with an aspect ratio of less than 100:1 for the ratio of longest to shortest dimensions when in a dry and uncompressed state.

5. The personal cleansing system according to any one of claims 1 to 4, wherein the liquid personal cleansing composition is aqueous.

6. The personal cleansing system according to any one of claims 1 to 5, wherein the aqueous cleansing composition containing core shell capsules comprises 3 to 95% by weight of one or more surfactants.

7. The personal cleansing system according to any one of claims 1 to 6, wherein the core shell capsules in the liquid personal cleansing composition have an average particle diameter from 1 µm to 500 µm diameter.

8. The personal cleansing system according to any one of claims 1 to 7, wherein the benefit agent in the core of the core shell capsules comprises 50% to 100% by weight of a perfume composition.

9. The personal cleansing system according to any one of claims 1 to 8, wherein the core shell capsules in the liquid personal cleansing composition are aminoplast core shell capsules.

10. A process for cleansing the skin comprising applying on the skin a liquid personal cleansing composition containing core shell capsules in which are encapsulated benefit agents and using a scrubbing device to release the benefit agents on the skin.

11. The use of a scrubbing device containing at least one hydrophobic synthetic polymer for breaking the capsules present in a liquid cleansing composition used to cleanse the skin while bathing or showering.
